# EUROPEAN PATENT SPECIFICATION

(11) **EP 1 234 183 B1**
(45) Date of publication and mention of the grant of the patent: **21.05.2008**
(21) Application number: 00988037.8
(22) Date of filing: 11.12.2000
(51) Int. Cl.: G01N 33/569, G01N 33/571

(54) **EIA FOR MONITORING LEGIONELLA PNEUMOPHILA IN WATER SAMPLES**
EIA ZUM ÜBERWACHEN VON LEGIONELLA PNEUMOPHILA IN WASSERPROBEN
EIA DESTINE AU CONTROLE DE LA PRESENCE DE LEGIONELLA PNEUMOPHILA DANS DES ECHANTILLONS D'EAU

(30) Priority: 10.12.1999 US 458998
(43) Date of publication of application: 28.08.2002
(73) Proprietor: Binax, Inc., Portland, ME 04103 (US)
(72) Inventor: MOORE, Norman, James, North Berwick, ME 03906 (US); WHIPKEY, Myron, David, Portland, ME 04103 (US); WELCH, James, William, Portland, ME 04103 (US)
(74) Representative: Becker Kurig Straus
(86) International application number: PCT/US2000/033586
(87) International publication number: WO 2001/042791

(56) References cited:
- EP-A- 0 119 893
- WO-A-00/10584
- US-A- 4 206 094
- US-A- 4 411 832
- US-A- 4 780 407
- US-A- 5 415 994
- HACKMAN BARBARA A ET AL: "Comparison of binax legionella urinary antigen EIA kit with binax RIA urinary antigen kit for detection of Legionella pneumophila serogroup 1 antigen" 1996, JOURNAL OF CLINICAL MICROBIOLOGY, VOL. 34, NR. 6, PAGE(S) 1579-1580 , XP002304544 ISSN: 0095-1137 * the whole document *
- AURELL HELENA ET AL: "Rapid detection and enumeration of Legionella pneumophila in hot water systems by solid-phase cytometry." APPLIED AND ENVIRONMENTAL MICROBIOLOGY, vol. 70, no. 3, March 2004 (2004-03), pages 1651-1657, XP002304543 ISSN: 0099-2240
- JURGENS ET AL.: 'Cross-reacting lipopolysaccharide antigens in legionella pneumophila serogroups 1 to 14' vol. 63, no. 6, June 1995, pages 2180 - 2184, XP002925153
- BARTHE ET AL.: 'Common epitope on the lipopolysaccharide of legionella pneumophila recognized by a monoclonal antibody' J. CLINICAL MICROBIOLOGY vol. 26, no. 5, May 1988, pages 1016 - 1023, XP002925154

## Description

### INTRODUCTION

This application relates to a test for detecting *Legionella pneumophila* in water samples which is useful, *inter alia,* for on-site monitoring of both essentially quiescent high sediment water, such as heating and air conditioning system cooling tower water and water that is moving through pipes or otherwise flowing, such as water that is, or is desired to be, rendered potable.

### BACKGROUND OF THIS INVENTION

The United States Occupational Safety and Health Agency ("OSHA") recommends that cooling tower water and other essentially still water having in the order of 1,000 colony-forming units or more ("CFU") per milliliter of *Legionella pneumonila* serogroup 1, the most common cause of human Legionnaires disease (also called Legionellosis), should be promptly treated to reduce this level substantially. Meanwhile OSHA also recommends that simultaneous medical surveillance and awareness training of building employees, building inhabitants and any other persons regularly in the building served by a cooling tower (if that is the source of the infected water) and all persons in frequent contact with the infected water source (in the case of other infected quiescent high sediment water) plus collection and monitoring of water samples from the infected source on a regular periodic basis be instituted. Assessment of past sick leave and illnesses of inhabitants and other personnel regularly exposed to the infected water, whether it is a building heating and cooling system or elsewhere, to see if any of them were infected with a *Legionella pneumophila*-caused illness is also recommended. OSHA further recommends that whenever the water available in a building, or water from another source used for drinking, washing and other domestic or public use, contains in the order of 100 CFU per ml. or more of *L. pneumophila* serogroup 1, treatment of the water to reduce the level of these bacteria markedly and all of the other measures described above should be promptly undertaken.

Unfortunately, the methodologies heretofore available for environmental monitoring of these water samples have been less than satisfactory. The bacteria in water samples can be cultured and the bacteria identified, but the procedure and the recognition of the specific bacteria grown in culture require highly trained, preferably experienced personnel -- and, moreover, as much as two weeks after sample collection may be needed before a culture result is obtainable. Obviously, such a test is not adequate to situations in which daily or several-times-a-day monitoring of the water is the desideratum. It is also less than satisfactory when there is reason to infer that a water source has become highly infected and that prompt remedial action is of high priority.

A direct fluorescence assay ("DFA") has been used to some extent for environmental monitoring of water samples, but the Centers for Disease Control have assessed this activity and have announced that use of DFA does not provide results adequate for effective environmental monitoring or evaluation of the *L. pneumophila* content of water.

WO 0010584 describes methods for detecting Legionella bacteria using specific antibodies against O-polysaccharides.

A polymeric chain reaction ("PCR") assay has been used in attempting to monitor *L*. *pneumophila* in water samples. This technique enables an assay result to be produced within the same day that the sample is collected, but it has two significant drawbacks -- namely, (1) the presence of rust in the sample (a frequent occurrence in cooling tower water samples and in water that has run through metal pipes in older buildings) can inhibit the accuracy and sensitivity of the test, and (2) the test is very difficult to run from a technical standpoint and hence has not been widely accepted.

It should also be noted that *L. pneumophila* present in building water supplies or building cooling towers are, in large part, whole bacteria in contrast to, *e.g., L. pneumophila* detectable in human urine of persons infected with Legionnaire's disease. These whole bacteria have not been subjected to the cell wall degradation that occurs naturally, *e.g*., in the human kidney and, accordingly, detection of their O-polysaccharide antigens, as described in the parent application for, *e.g*., urine samples, is a more difficult problem. It should further be recognized that the likelihood is that some nonviable, and some living, bacteria are likely to be present in infected water samples. The tests available, including those herein described, do not distinguish between the viable and nonviable bacteria present.

The need for a rapid test for *L. pneumophila* serogroup 1 that is of high sensitivity and accuracy and can be used, preferably on-site, to monitor *L. pneumophila* serogroup 1 levels, *e.g.,* in water supplies of buildings and water from building cooling towers has accordingly been a grave one.

### BRIEF DESCRIPTION OF THE INVENTION

To meet this need, applicants turned first, as more fully described below, to the ICT assay for *L. pneumophila* serogroup 1 that is described in detail in the parent application, which produces an assay result within approximately 15 minutes of sample application and is well adapted to on-site use because untrained personnel can use it easily. In essence, applicants found this test to be somewhat useful, but limited by the lower level of sensitivity, specifically somewhere between 500 and 1,000 CFU of *L. pneumophila* serogroup 1 present per ml. per liter of water sampled, that it exhibits with water samples in comparison to its much more acute sensitivity displayed when used to detect *L. pneumophila* in mammalian fluids, *e.g.,* human urine. Applicants then developed a modified enzyme immunoassay ("EIA") using a coated tube in which *L. pneumophila* serogroup 1 raw polyclonal antibodies that have first been purified according to the affinity purification procedure described and claimed in the parent application are first used to coat tubes. The assay, which can produce a test result within an hour from sampling, is run by introducing sample and an enzyme conjugate of the same affinity purified and antigen-specific polyclonal antibodies to the coated tube, followed by an incubation period of at least about 20 minutes, color development and assessment of the result. This test was found to be capable, on a highly reproducible basis, of detecting 50 CFU per ml./liter of water sampled, or 5 x 10⁴ CFU total introduced into the test tube of *L*. *pneumophila* serogroup 1. When further modified by extending the incubation period to 60 minutes and reading after 5 minutes' color development in a spectrophotometer at 450 nm absorbance, the same test detected 5 CFU per ml. per liter of water sampled, or 5 x 10³ CFU total per test *of L. pneumophila* serogroup 1.

In each of the ICT and EIA assays described herein, the use of the antigen-specific antibodies obtained by affinity purifying raw polyclonal antibodies to *L. pneumophila* serogroup 1 is critical to the test performance attained. In contrast, substituting unpurified raw polyclonal antibodies to *L. pneumophila* serogroup 1, when used in either the ICT test or the EIA test, gave background of such magnitude that differentiating the assay result with test sample against a blank run cannot be achieved without the use of complex laboratory instrumentation and skilled personnel to run it -- neither of which is compatible with rapid on-site initial testing and subsequent monitoring.

### DESCRIPTION OF THE DRAWINGS

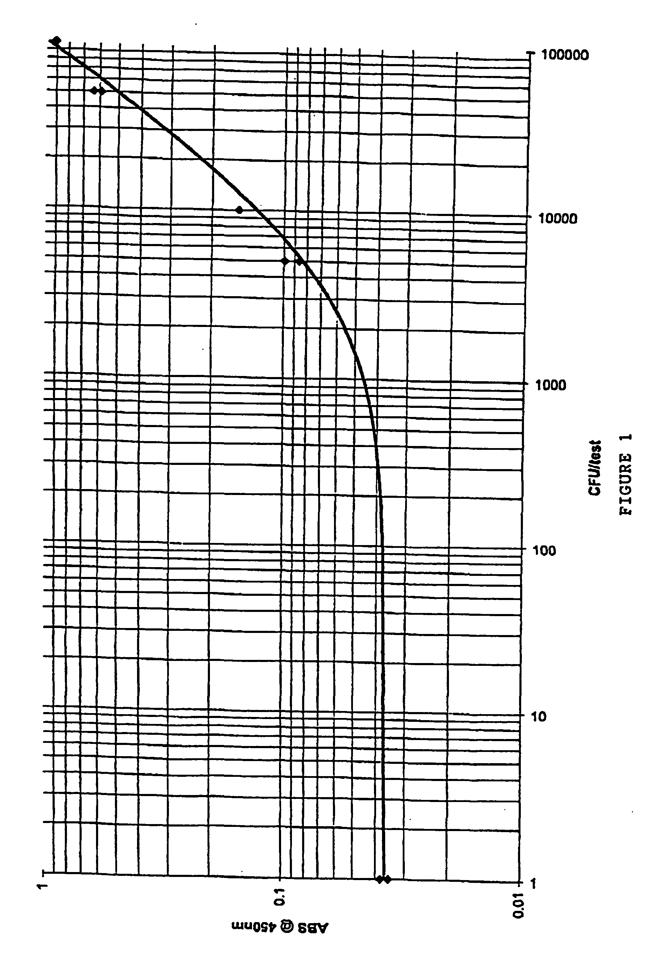

Figure 1 is a graph of data collected in running Example 3, a quantitative test.

### DETAILED DESCRIPTION OF THE INVENTION

The affinity purified antigen-specific antibodies to *L. pneumophila* serotype 1 are disclosed in the parent application, with detailed examples showing the preferred method for purifying them.

The ICT device preferred and its preparation are likewise described in the parent application in detail, especially in Example VII thereof.

A notable difference between the specific assay procedure described in the parent application and that found effective in assaying both quiescent, high sediment water -- here specifically cooling tower water samples -- and low sediment, flowing water samples which in this instance were building water supply samples, is that a substantial volume of each water sample should be subjected to a pre-assay step to concentrate the amount of antigen present. This step may be a filtration step through a fine pore filter capable of retaining *L. pneumophila* on its surface, whereupon the sample for assay is obtained by swabbing the material retained on the filter with a swab comprising a handle and an affixed pad fashioned, preferably, from a fibrous material. In lieu of a fibrous material, the swab pad may be made from a foamed open pore material.

To obtain a sample of normally flowing, low sediment water, such as the water supply available from the faucet taps of a building, including a household, the volume of water to be pre-filtered in preparation for assay is at least 1,000 ml. Water from cooling tower sources and other essentially quiescent waters having high sediment loads -- *e.g.,* water from a quiescent pond or pool -- will normally provide a filter residue sufficient for assay from a 100 ml. cut. The filter preferred by applicants is the Gelman GN-6 Metricel^{®} 47 mm. grid filter having a pore size of 0.45 µm, but other filters of the same or smaller pore size, *e.g*., 0.22 µm, may be used.

In lieu of filtering, the water sample may be centrifuged at high speed and the sample remaining after decanting or aspirating off the water may be transferred to the device.

Another alternative to both filtering and centrifugation that is known in the art is the addition to the sample of an aqueous solution of antibody-coated, extremely finely divided magnetic particles. These particles tend to draw the antigen from the sample so it reacts with the antibodies on their surface. When a local magnetic field is applied, they are magnetically attracted toward one another and form a coherent mass, from which the water can be aspirated or decanted. When this form of immunoconcentration is used, one of ordinary skill in immunology, applying techniques well known in the art, can readily design a specific EIA procedure that employs the antigen-specific anti-*L*. *pneumophila* serogroup 1 antibodies of this invention, to provide a satisfactory qualitative or quantitative assay result.

The preparation of the coated tubes for the modified EIA test may be performed according to any conventional protocol for coating such tubes. Applicants' preferred method is described in Example 2 hereof, below.

Example 2 describes how to run a sandwich assay, but those of ordinary skill in immunology will readily perceive that the test procedure can be modified by the use of ordinary skill so as to conduct a competition assay. Furthermore, the sandwich assay described in Example 2 may readily be modified to be run in a "forward flow" rather than a simultaneous fashion as described in Example 2 so that the sample is added to the tubes and incubated with the antibodies for the desired time, the tube is washed and antibody-enzyme conjugate is then added, allowed to react for a desired period of time, followed by further washing and color development.

It should further be noted that the incubation time of 20 minutes for tube plus sample plus antibody-enzyme conjugate set forth in Example 2 is the minimum time necessary to achieve a satisfactory assay and that even much longer incubation times can be used without departing from this invention.

Similarly the EIA can be designed to be run on microtiter plates which may be coated with antibodies, or in any other known manner.

The ensuing examples serve to illustrate the performance of the ICT and EIA tests for *L. pneumophila* serogroup 1 on environmental water samples. It is to be understood that tests for other *Legionella pneumophila* serotypes and other *Legionella* bacteria may be analogously designed and will be analogously conducted, with the substitution of the antigen-specific antibodies for the appropriate species or serotype of a species of *Legionella,* affinity purified according to the methods described in the parent application.

It should be noted that both the ICT test of Example 1 and the EIA test of Example 2 have been designed as qualitative tests to permit ready use in on-site monitoring and to allow on-site personnel to judge from color appearance alone whether an ongoing water treatment is effective to reduce *L. pneumophila* serogroup 1 presence to a safe level or whether, where treatment is not yet in progress, initiation of treatment is advisable. A quantitative EIA assay run in the same coated tubes used in Example 2 is described in Example 3 and Figure 1 shows its results. As those of ordinary skill in immunochemistry will readily recognize, tests run in accordance with other well known quantitative techniques, such as providing color intensity standards keyed to the number of antigens of *L. pneumophila* serogroup 1 present per ml./per liter of water sampled, could readily be applied. This number of antigens includes those from whole bacteria, whether viable or nonviable, and those separated from bacterial cells. As is also familiar to those of ordinary skill in immunochemistry, color development in the EIA can be stopped with addition of HCl or other strong acid and color intensity can be read instrumentally as in Example 3.

### Example 1 -- ICT Test

### A. Preparation of Test Device

The ICT device is generally described, and also depicted in drawings, in the parent application. Its preparation, including the preparation and construction of the test strip, is described in Example VII of the parent application.

### B. Immunoassay Procedure

Several samples of water were run on identically prepared ICT devices. The first such sample was tap water to which *Legionella pneumophila* serogroup 1 bacteria obtained originally from Centers for Disease Control and grown in culture had been added up to a level of about 50 CFU per ml. Initially, 1,000 ml. of this water was put through a filtration unit having a Gelman GN-6 Metricel^{®} grid filter of 0.45 µm pore size and a 47 mm. diameter. The filtrate was discarded. The sample was collected from the material on the filter using a swab with a fibrous Dacron pad which was stroked thoroughly across the surface of the filter. The swab was then inserted into the device in the manner described in Example VIII of the parent application. Six drops of "Reagent A" -- in this instance a solution of .5 M Tris base containing 2 percent of SB3-8, a commercially available Zwitterionic detergent from Sigma Chemical Co. was added to the swab. This Reagent A has a pH of 8.0 ± 0.1. This Reagent A has the multiple purpose of dissolving and inducing flow of the sample and assisting cell wall breakdown of the bacteria to ensure accessibility of their antigens to the antibodies on the test strip. The device was closed and the sample flowed along the conjugate pad. After 15 minutes the test sample and control lines were viewed in the window. No color was observed, showing the sample to be outside the capability of the test to detect.

By inoculating tap water with the same *L. pneumophila* serogroup 1 bacteria at successively higher levels of 100 CFU per ml., 500 CFU per ml. and 1,000 CFU per ml. per liter of water sample, and pre-filtering 1,000 ml. of water containing each concentration, collecting a sample from the filter in the identical manner and running each of these samples in the ICT device in exactly the same manner as described in the preceding paragraph, it was determined that no positive sample line could be detected at 100 or 500 CFU per ml. of target bacteria per liter of water sample, but a positive sample line definitely appeared in the sample containing 1,000 CFU per ml. per liter of the *L. pneumophila* bacteria. Accordingly, it was concluded that the sensitivity of the ICT test for *L. pneumophila* serogroup 1 in tap water is between 500 CFU and 1,000 CFU per ml., per liter of water sampled.

This conclusion was confirmed by running the same ICT test on cooling tower waters at various *L. pneumophila* serogroup 1 CFU levels, in a manner in all respects identical, except for the volume of water initially filtered, which on these high sediment waters was 100 ml. rather than 1,000 ml.

ICT devices were prepared using raw, untreated polyvalent antibodies to *L. pneumophila* serogroup 1 and a gold conjugate of such antibodies in lieu, respectively, of antigen-specific antibodies purified as disclosed in the parent application and their gold conjugate in the corresponding portions of the test strip.

Several tests were each run in the identical manner described, using tap water and cooling tower water containing no added *L. pneumophila* bacteria. In each of the ICT tests, a faint sample line was observed, demonstrating that raw polyclonal antibodies cross react with other substances present in most water samples, including benign non-*Legionella* bacteria, to an extent that would render them essentially unusable to distinguish *L. pneumophila* serogroup 1 from other substances present in water in an ICT test.

### Example 2 -- EIA Test

This example describes Applicants' preferred embodiment of the modified EIA assay for on-site testing of water samples for *L. pneumophila* serogroup 1. As already discussed to some extent and as those of ordinary skill in immunology will readily perceive, this assay can be designed to operate in numerous modes that are well known in the art, using various devices such as coated solid inserts or coated beads in lieu of coated tubes and using other enzymes and chromogenic reagents, or by using chemiluminescent or bioluminescent tags plus an instrument to read the result. So long as such other modes employ the antigen-specific antibodies of this invention, produced by the affinity purification process as described in the parent application, employing the O-polysaccharide antigen described in that application, they are within the scope of this invention.

### A. Preparation of Coated Tubes

Nunc Star tubes were coated with the antigen-specific affinity purified antibodies to *L*. *pneumophila* serogroup 1 antigen which are described in the parent case. To effect the coating, these antibodies in the amount of 5.0 µg/ml. were added to an aqueous coating solution of pH 7.0 containing NaH₂PO₄ (14.5 g./liter), Na₂HPO₄ (11.77 g./liter) and glutaraldehyde (25% by wt.) in water (0.2 ml. per liter). After mixing, 200 µl of this solution was added to each Nunc tube and the tubes were incubated overnight at room temperature.

The following morning, the solution was decanted from the tubes and to each, 4 ml. of a glycine-bovine serum albumen (BSA) solution was added, followed by incubation of at least one hour and up to four hours at room temperature. This latter solution contained 7.5 g./liter of glycine and 5 ml./liter of 10% BSA and had a pH of 7.4 ± 0.1. Following the incubation, this solution was decanted from the tubes and 200 µl per tube of a solution of pH 7.4 ± 0.1 containing 40 g./liter of sucrose and 100 ml./liter of BSA was added. The tubes were again incubated overnight. The following morning the sucrose-BSA solution was removed by aspiration from each tube, and the tubes were inverted and left in a dry room for at least 36 hours. Each tube so prepared had a coating of 1.0 µg of antibody. In other work, it was determined that the optimum coating range is from 0.05 µg to 5.0 µg per tube and that at least 0.05 µg per test of antibody must be used.

### B. Conduct of the EIA Test

For these tests, the water to be tested was filtered in the same way as is described for the ICT tests.

For each test, there is added to a coated tube prepared as described in Example 2A, 200 µl of a buffer composed of aqueous .05 M Tris HCl with 2 to 5 percent of Tween-20 having a pH of 7.0 ± 0.1. Approximately 200 µl of a conjugate of horseradish peroxidase (HRP) and anti*-Legionella pneumophila* serotype 1 antigen-specific antibodies (affinity purified as described in the parent application) is added. A swab with a swab pad of fibrous Dacron is thoroughly stroked across the filter surface and is then placed into the tube and twirled. The tube containing the swab is then incubated for 20 minutes at room temperature, whereupon the liquid is pressed from the swab and left in the tube, while the swab is removed and discarded. Approximately 100 µl of wash solution of 0.05 M Tris-HCl (also containing 9.0 percent of Triton X-100) are added to the tube, and the tube is then filled with tap water or deionized water. The liquid in the tube is thereupon decanted. This wash procedure is repeated four times with tap or deionized water to remove any unbound conjugate. Following the fifth wash and decantation, approximately 200 µl of a tetramethylbenzidine/peroxide mixture (K-Blue obtained from Neogen) is added and the tube is allowed to stand for two minutes. The presence of any blue color in the tube after that period indicates the presence of *L. pneumophila* serogroup 1; the absence of color indicates the sample is presumptively negative for *L*. *pneumophila* serogroup 1.

In other work it was determined that the *amount* of conjugate added in this test should be between .02µg and 0.2µg.

By conducting a series of EIA tests in coated tubes in the manner just described on water samples of the same two types employed in Example 1B, containing in each instance varying known levels of *L. pneumophila,* it was established that this test can detect as little as 50 CFU per ml/per liter of sample water of *L. pneumophila* serogroup 1 per liter of water on a repeatable and consistent basis.

For comparison purposes tubes were coated in the manner described in Example 1A with raw polyvalent antibodies to *L. pneumophila* serogroup 1 and the assay was run in them on both tap water and cooling tower water containing no added *L. pneumophila* serogroup 1. In each instance, these raw antibodies cross-reacted with other substances present to produce a bluish tinge in the tube, again demonstrating that raw polyclonal antibodies produce an amount of background that renders them infeasible as a reagent for detecting *L. pneumophila* serotype 1 in water samples suspected of harboring these bacteria.

### Example 3

A series of quantitative EIA's for *L. pneumophila* serogroup 1 were run in the same coated tubes described in Example 2, with a longer incubation time and color development period, followed by reading the absorbance at 450 nm of each tube using a Beckmann spectrophotometer.

The samples for these runs were prepared by adding to tap water incremental amounts of 5 x 10³, 1 x 10⁴, 5 x 10⁴ and 1 x 10⁵ CFU per test of cultured *L. pneumophila* serogroup 1 that had first been rendered nonviable with formalin. Duplicate runs were made at the 5 x 10³ and 5 x 10⁴ levels. Two blank runs were made with no bacteria (and hence no antigen) present.

In all cases, the conjugate, assay buffer and wash solution were the same, and their amounts were the same, as in Example 2. The incubation time for the coated tube containing assay and buffer was 60 minutes in each run. After the tubes were thoroughly washed as described in Example 2, K-Blue was added to each tube in the amount described in Example 2. The tubes were allowed to stand for 5 minutes at ambient temperature, whereupon 1 ml. of 1 N H₂SO₄ was added to each tube to stop color development. The absorbance at 450 nm of each tube was then read in the spectrophotometer.

The absorbances were graphed vs. "CFU/test" and the graph is shown in Figure 1. This assay was 10 times more sensitive than the qualitative tests of Example 2. It detected 5 x 10³ CFU of antigen per test at an absorbance value 2.5 times higher than the blank, as shown in Figure 1. "5 x 10³ CFU" per test as used here corresponds to 5 CFU antigen/ml./per liter of water sampled.

Clearly, increased incubation time, increased color development time and/or quantification of the assay using the spectrophotometer improved the test sensitivity. Whether the qualitative test can be made more sensitive, *e.g*., by lengthened incubation time, is being investigated. It is clear that, for most on-site evaluation and monitoring, the use of the spectrophotometer is infeasible. At the same time, it appears that further development work may produce a more sensitive qualitative test.

Those of ordinary skill in immunology will readily perceive that numerous forms of enzyme immunoassay may be devised and utilized without departing from the scope of this invention. Furthermore, the concentration step described herein may be omitted in instances where the sample for analysis is not dissolved or dispersed in water -- such as, *e.g*., where it is clear that biofilm on the inner surface of a faucet, scum on the inner surface of a cistern cover or solids floating on a still pool of water -- should be assayed for the presence of *L*. *pneumophila* serotype 1, a swab may be rubbed over the surface and then used to deliver sample directly to an assay device.

Because it is known that enzyme immunoassays can be developed in so many forms and combinations and with so many variations from one another and from the specific EIA's of Examples 2 and 3 without departing from the scope of the present invention, it is intended that the invention be limited only by the ensuing claims.

## Claims

1. An enzyme immunoassay of the sandwich type for the detection of L. pneumophila in environmental water in which the detecting agents are antigen-specific antibodies obtained by purifying raw polyvalent anti-Legionella pneumophila antibodies on a chromatographic column to which is coupled a conjugate of a protein free polysaccharide antigen of L. pneumophila and a spacer molecule, wherein the enzyme is horse radish peroxidase, the assay is conducted in a tube coated with the antigen-specific antibodies and the sample is incubated with the antigen specific antibodies for at least 20 minutes.

2. An enzyme immunoassay according to claim 1 in which a pre-assay antigen concentration step is first performed on the water sample.

3. An enzyme immunoassay according to claim 2 in which the concentration step is filtration or centrifugation of at least 100 ml. of water and it is followed by rubbing the pad end of the swab over the surface on which the antigen has been concentrated, and delivering the material collected by the swab to the assay.

4. An enzyme immunoassay according to claim 2 wherein the concentration step comprises mixing at least 100 ml. of water with an aqueous solution of finely divided magnetic particles which have been precoated with the antigen-specific antibody of claim 1 and the resulting antibody-antigen product is subjected to a modified EIA procedure.

5. An enzyme immunoassay according to claim 1 in which the antigen-specific antibodies are present in an amount between 0.05 µg per test and 5.0 µg per test.

6. An enzyme immunoassay according to claim 1 wherein at least 0.05 µg of antigen-specific antibodies must be used in each test.

7. An enzyme immunoassay according to claim 1 in which the bacterium to be detected is L. pneumophila serogroup 1.

## Patentansprüche

1. Enzymimmunoassay vom Sandwich-Typ zum Nachweis von L. pneumophila in Umgebungswasser, wobei die nachweisenden Mittel antigenspezifische Antikörper sind, welche durch Reinigen roher polyvalenter anti-Legionella pneumophila Antikörper auf einer Chromatographiesäule, an die ein Konjugat eines proteinfreien Polysaccharidantigens von L. pneumophila und eines Abstandhaltermoleküls gekoppelt ist, erhalten werden, wobei das Enzym Meerrettichperoxidase ist, der Assay in einem Röhrchen durchgeführt wird, das mit den antigenspezifischen Antikörpern beschichtet ist und die Probe mit den antigenspezifischen Antikörpern für mindestens 20 Minuten inkubiert wird.

2. Enzymimmunoassay nach Anspruch 1, wobei in der Wasserprobe vor dem Assay zunächst ein Antigen-Konzentrierungsschritt durchgeführt wird.

3. Enzymimmunoassay nach Anspruch 2, wobei der Konzentrierungsschritt eine Filtration oder eine Zentrifugation von mindestens 100 ml Wasser ist, und gefolgt wird von einem Reiben des Auflageendes des Tupfers über die Oberfläche auf der das Antigen konzentriert wurde, und wobei das durch den Tupfer gesammelte Material an den Assay abgegeben wird.

4. Enzymimmunoassay nach Anspruch 2, wobei der Konzentrierungsschritt ein Mischen von mindestens 100 ml Wasser mit einer wässrigen Lösung von fein zerteilten Magnetteilchen umfasst, die mit dem antigenspezifischen Antikörper nach Anspruch 1 vorbeschichtet sind, und wobei das so erhaltene Antikörper-Antigen-Produkt einem modifizierten EIA-Verfahren unterzogen wird.

5. Enzymimmunoassay nach Anspruch 1, wobei die antigenspezifischen Antikörper in einer Menge zwischen 0,05 µg pro Test und 5,0 µg pro Test vorliegen.

6. Enzymimmunoassay nach Anspruch 1, wobei mindestens 0,05 µg der antigenspezifischen Antikörper in jedem Test verwendet werden müssen.

7. Enzymimmunoassay nach Anspruch 1, wobei das nachzuweisende Bakterium L. pneumophila der Serogruppe 1 ist.

## Revendications

1. Un test immuno-enzymatique du type sandwich pour la détection de L. pneumophila dans de l'eau du milieu environnant, dans lequel les agents de détection sont des anticorps spécifiques d'antigène obtenus en purifiant des anticorps polyvalents anti-Legionella pneumophila bruts sur une colonne chromatographique à laquelle est couplé un conjugué d'un antigène de polysaccharide de L. pneumophila exempt de protéine et d'une molécule d'espacement, dans lequel l'enzyme est la peroxydase de raifort, le test est effectué dans un tube enduit avec des anticorps spécifiques d'antigène et l'échantillon est incubé avec les anticorps spécifiques d'antigène pour au moins 20 minutes.

2. Un test immuno-enzymatique selon la revendication 1 dans lequel une étape de concentration d'antigène de pré-test est premièrement effectuée sur l'échantillon d'eau.

3. Un test immuno-enzymatique selon la revendication 2 dans lequel l'étape de concentration est la filtration ou centrifugation d'au moins 100 ml d'eau, suivie par le frottement de l'extrémité du coussinet du tampon sur la surface sur laquelle l'antigène a été concentré, et la remise au test du matériel récolté par le tampon.

4. Un test immuno-enzymatique selon la revendication 2 dans lequel l'étape de concentration comprend le mélange d'au moins 100 ml d'eau avec une solution aqueuse de particules magnétiques finement divisées qui ont été préalablement enduites avec l'anticorps spécifique d'antigène de la revendication 1 et le produit antigène-anticorps résultant est soumis à un procédé EIA modifié.

5. Un test immuno-enzymatique selon la revendication 1 dans lequel les anticorps spécifiques d'antigène sont présents en une quantité entre 0.05 µg par test et 5.0 µg par test.

6. Un test immuno-enzymatique selon la revendication 1 dans lequel au moins 0.05 µg d'anticorps spécifiques d'antigène doit être utilisé dans chaque test.

7. Un test immuno-enzymatique selon la revendication 1 dans lequel la bactérie à détecter est L. pneumophila sérotype 1.
